(19) Europäisches Patentamt
European Patent Office
Office européen des brevets

(11) Veröffentlichungsnummer: **0 351 351**
**A2**

# (12) EUROPÄISCHE PATENTANMELDUNG

(21) Anmeldenummer: **89730163.6**

(22) Anmeldetag: **14.07.89**

(51) Int. Cl.5: **C 07 D 457/12**
A 61 K 31/48

(30) Priorität: **15.07.88 DE 3824659**

(43) Veröffentlichungstag der Anmeldung:
**17.01.90 Patentblatt 90/03**

(84) Benannte Vertragsstaaten:
**AT BE CH DE ES FR GB GR IT LI LU NL SE**

(71) Anmelder: **SCHERING AKTIENGESELLSCHAFT Berlin und Bergkamen**
**Müllerstrasse 170/178 Postfach 65 03 11**
**D-1000 Berlin 65 (DE)**

(72) Erfinder: **Heindl, Josef, Dr.**
**Eitel-Fritz-Strasse 40**
**D-1000 Berlin 38 (DE)**

**Sauer, Gerhard, Dr.**
**Königsbacher Zeile 41A**
**D-1000 Berlin 28 (DE)**

**Wachtel, Helmut, Dr.**
**Suarezstrasse 22**
**D-1000 Berlin 19 (DE)**

## (54) 2'-, 12'- oder 13'-substituierte 3-(8'alpha-Ergoninyl)-1,1-diethylharnstoffe, ihre Herstellung und Verwendung in Arzneimitteln.

(57) Es wird ein Verfahren zur Herstellung von 2'-, 12'- oder 13'-substituierten 3-(8'α-Ergolinyl)-1,1-diethylharnstoffen der allgemeinen Formel I und deren Säureadditionssalzen

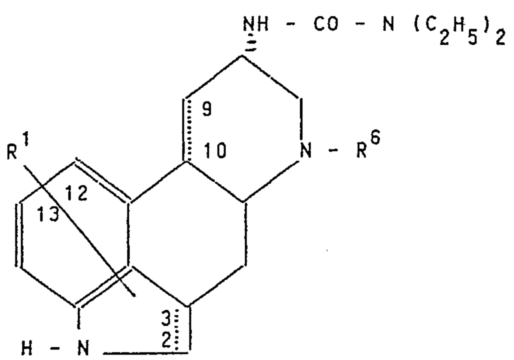

I

worin
$R^1$ $C_{1-10}$-Alkyl, $C_{2-10}$-Alkenyl, $C_{2-10}$-Alkinyl, gegebenenfalls substituiertes Phenyl oder Hetaryl bedeutet,
$R^6$ $C_{1-4}$-Alkyl und
$C_2$- -$C_3$ und $C_9$- -$C_{10}$ eine Einfach oder Doppelbindung bedeuten und $C_2$- -$C_3$ eine Doppelbindung ist, wenn $R^1$ in 2-Stellung steht, beschrieben sowie die neuen Verbindungen der allgemeinen Formel Ia

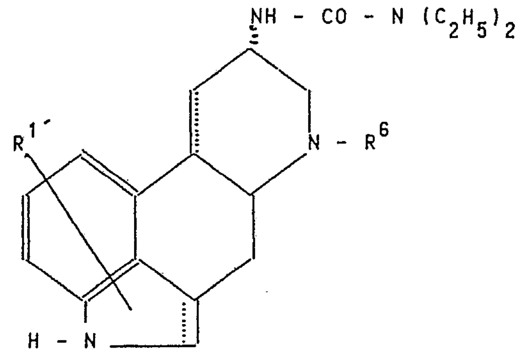

Ia

und deren Verwendung in Arzneimitteln.

EP 0 351 351 A2

## Beschreibung

**2′-, 12′- oder 13′-substituierte 3-(8′α-Ergolinyl)-1,1-diethylharnstoffe ihre Herstellung und Verwendung in Arzneimitteln**

Die Erfindung betrifft 2′-, 12′- oder 13′-substituierte 3-(8′α-Ergolinyl)-1,1-diethylharnstoffe, ihre Herstellung und Verwendung in Arzneimitteln.

Die Herstellung von 2-, 12- oder 13-substituierten Ergolinen wird beispielsweise in der EP-A 160842 und EP-A 220129 beschrieben. Diese Synthesen gehen von einem Halogenergolinylharnstoffderivat aus, das entweder nach Überführung in die Lithiumverbindung anschließend einem elektrophilen Austausch unterworfen wird oder unter Palladium-Katalyse in Gegenwart einer Base in ein substituiertes Alkenyl- oder Alkinyl-Derivat überführt wird.

Nach dem erfindungsgemäßen Verfahren können die gewünschten Endprodukte im Gegensatz zu den bekannten zweistufigen Verfahren in einer Stufe erhalten werden, wobei jeder beliebige organische Rest durch die Umsetzung mit der metallorganischen Verbindung eingeführt werden kann.

Das beanspruchte Verfahren stellt daher ein einstufiges Verfahren dar, das in sehr guten Ausbeuten zu pharmakologisch wertvollen Verbindungen führt.

Die Erfindung betrifft das Verfahren zur Herstellung von 2′-, 12′- oder 13′-substituierten 3-(8′α-Ergolinyl)-1,1-diethylharnstoffen und deren Säureadditionssalzen der allgemeinen Formel I

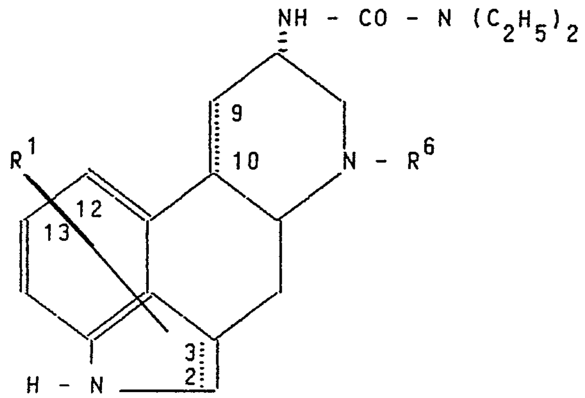

I

worin

$R^1$ $C_{1-10}$-Alkyl, $C_{2-10}$-Alkenyl, $C_{2-10}$-Alkinyl, gegebenenfalls substituiertes Phenyl oder Hetaryl

$R^6$ $C_{1-4}$-Alkyl,

$C_2$- -$C_3$ und $C_9$- -$C_{10}$ eine Einfach- oder Doppelbindung und, wenn $R^1$ in 2-Stellung steht, $C_2$- -$C_3$ eine Doppelbindung bedeutet, dadurch gekennzeichnet, daß man eine Verbindung der allgemeinen Formel II

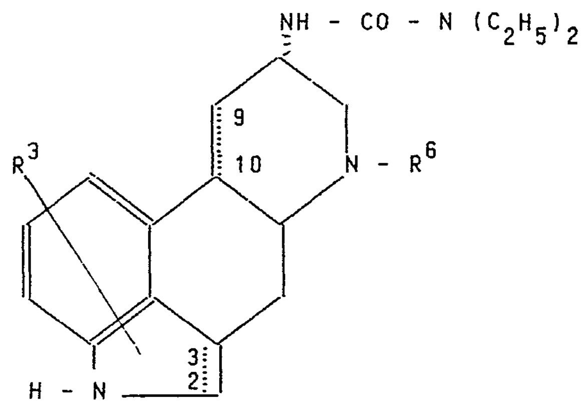

II

worin

$C_2$- -$C_3$, $C_9$- -$C_{10}$ und $R^6$ die obige Bedeutung haben und $R^3$ Halogen ist, in Gegenwart eines Palladium- oder Nickelkatalysators umsetzt mit einer metallorganischen Verbindung der allgemeinen Formel III

$R^1$ - Me - $X_n$ III

worin

$R^1$ die obige Bedeutung hat,

Me ein Metallatom,

X Halogen, Hydroxy oder $C_{1-4}$-Alkyl und

n 1 bis 3 bedeutet und gegebenenfalls anschließend die Säureadditionssalze bildet.

Stellt $C_2$- -$C_3$ eine Einfachbindung dar, so steht das Wasserstoffatom in 3-Stellung β-ständig, stellt $C_9$- -$C_{10}$ eine Einfachbindung dar, so steht das Wasserstoffatom in 10-Stellung α-ständig.

Unter $C_{1-10}$-Alkyl ist jeweils ein geradkettiger oder verzweigter Alkylrest zu verstehen, vorzugsweise ein niederer Alkylrest mit 1 bis 4 Kohlenstoffatomen, wie beispielsweise Methyl, Ethyl, Propyl, Isopropyl, Butyl, Isobutyl, sek. Butyl, tert. Butyl, Pentyl, Hexyl, Heptyl, Octyl, Nonyl, 1,2-Dimethylheptyl, Decyl, 2,2-Dimethylpropyl, 2-Methylbutyl, 3-Methylbutyl u.a.

Der Alkenylrest $R^1$ hat jeweils insbesondere 2 bis 4 Kohlenstoffatome wie beispielsweise Vinyl, 1-Propenyl, 2-Propenyl-, 3-Methyl-2-propenyl, 1-Butenyl, Methallyl u.a. Als Alkinylrest $R^1$ sind insbesondere Ethinyl, 1-Propinyl, 2-Propinyl mit 2 bis 4 Kohlenstoffatomen geeignet.

Als Hetarylrest kommen 5- oder 6-gliedrige Heteroaromaten in Betracht, die 1 bis 2 Heteroatome wie Stickstoff, Sauerstoff und/oder Schwefel enthalten können.

Insbesondere geeignet sind 5-6-gliedrige Heteroaromaten mit einem Stickstoff-, Schwefel- oder Sauerstoffatom und 5-6-gliedrige stickstoffhaltige Heteroaromaten, die noch ein weiteres Sauerstoff-, Schwefel- oder Stickstoffatom enthalten wie beispielsweise Furan, Thiophen, Pyrrol, Pyridin, Pyrimidin, Pyrazin, Pyridazin, Thiazol, Imidazol und Oxazol.

Der Phenylrest kann mono- oder disubstituiert sein in o-, m- oder p-Stellung beispielsweise durch Halogen wie Fluor, Chlor und Brom, $C_{1-4}$-Alkyl, $C_{1-4}$-Alkoxy Trifluormethyl, $NO_2$ oder Formyl.

Als Halogen $R^3$ ist Fluor, Chlor und insbesondere Brom und Jod geeignet. Als Halogen X kommen insbesondere Chlor und Brom in Betracht.

Geeignete Nickel- und Palladiumkatalysatoren sind beispielsweise 1,3-Diphenylphosphinopropan-nickel-II-chlorid, Bis-tri-o-tolylphosphin-palladium-II-chlorid, Bis-triphenylphosphin-palladium-II-chlorid, Tetrakis-triphenylphosphin-palladium-II-chlorid und 1,1′-Bis-diphenylphosphinoferrocen-palladium-II-chlorid.

Die metallorganische Verbindung der allgemeinen Formel III kann als Metallatom Magnesium, Zink, Zinn oder Bor enthalten, wobei der Substituent X jeweils nach der Wertigkeit des Metallatoms ein- bis dreifach stehen kann.

Die erfindungsgemäße Umsetzung kann in einem geeigneten inerten Lösungsmittel gegebenenfalls auch als Suspension, durchgeführt werden. Für die Umsetzung geeignet sind beispielsweise cyclische oder acyclische Ether wie Diethylether, Tetrahydrofuran und Dioxan, Kohlenwasserstoffe wie Toluol und Benzol, Dimethylformamid u.a.

Die Reaktion wird bei Temperaturen von 0 °C bis zur Siedetemperatur des Reaktionsgemisches durchgeführt.

Nach dem erfindungsgemäßen Verfahren können sowohl die bekannten 2-Alkyl-, Alkenyl- und Alkinyl-Derivate und 12- und 13-Alkyl-Derivate wie auch die neuen Ergolinylharnstoffderivate der allgemeinen Formel Ia

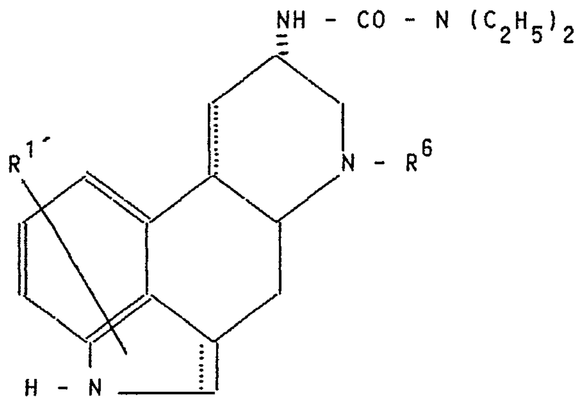

Ia

worin

$C_2$- -$C_3$, $C_9$- -$C_{10}$ und $R^6$ die obige Bedeutung haben und $R^1$ in 12- und 13-Stellung die Bedeutung von $R^{1'}$ gemäß der allgemeinen Formel I mit Ausnahme von $C_{1-10}$-Alkyl hat und $R^{1'}$ in 2-Stellung gegebenenfalls substituiertes Phenyl oder Hetaryl bedeutet und deren Säureadditionssalze, hergestellt werden.

Die physiologisch verträglichen Säureadditionssalze leiten sich von bekannten organischen und anorganischen Säuren ab wie beispielsweise Salzsäure, Schwefelsäure, Bromwasserstoffsäure, Maleinsäure, Fumarsäure, Zitronensäure, Weinsäure.

Die Überführung in die physiologisch verträglichen Säureadditionssalze kann nach den üblichen Methoden erfolgen. Beispielsweise werden zur Bildung von Salzen die Verbindungen der Formel I in wenig Methanol oder Methylenchlorid gelöst und mit einer konzentrierten Lösung der gewünschten Säure versetzt.

Die Verbindungen der allgemeinen Formel Ia weisen im Tierversuch interessante pharmakodynamische Eigenschaften auf und können daher als Arzneimittel verwendet werden. Insbesondere besitzen die erfindungsgemäßen Verbindungen der allgemeinen Formel Ia, die in 12- oder 13-Stellung substituiert sind, dopaminagonistische und die in 2-Stellung substituiert sind, dopaminantagonistische Wirkung und können daher als Dopaminagonisten beispielsweise zur Behandlung des Morbus Parkinson und auf Grund des Dopaminantagonismus als Neuroleptika zum Beispiel bei der Behandlung der Schizophrenie verwendet werden.

Zur Verwendung der erfindungsgemäßen Verbindungen als Arzneimittel werden diese in die Form eines pharmazeutischen Präparats gebracht, das neben dem Wirkstoff für die enterale oder parenterale Applikation

geeignete pharmazeutische, organische oder anorganische inerte Trägermaterialien, wie zum Beispiel Wasser, Gelatine, Gummi arabicum, Milchzucker, Stärke, Magnesiumstearat, Talk, pflanzliche Öle, Polyalkylenglykole usw. enthält. Die pharmazeutischen Präparate können in fester Form, zum Beispiel als Tabletten, Dragees, Suppositorien, Kapseln oder in flüssiger Form, zum Beispiel als Lösungen, Suspensionen oder Emulsionen vorliegen. Gegebenenfalls enthalten sie darüberhinaus Hilfsstoffe wie Konservierungs-, Stabilisierungs-, Netzmittel oder Emulgatoren, Salze zur Veränderung des osmotischen Drucks oder Puffer.

Die nachfolgenden Beispiele sollen das erfindungsgemäße Verfahren erläutern.

**Beispiel 1**

300 mg (0,72 mMol) 3-(12-Brom-6-methyl-8α-ergolinyl)-1,1-diethylharnstoff werden in 1,5 ml Toluol suspendiert, mit 16 mg (0,022 mMol) Bis-(tri-o-tolylphosphin)-palladium-II-chlorid und 298 mg (0,94 mMol) Tributylvinylzinn versetzt und die Mischung unter Rühren 3 Stunden auf 100 °C erhitzt. Die Reaktionsmischung wird auf Eis gegossen, mit 25 %iger Ammoniaklösung alkalisch gestellt, mit Dichlormethan ausgeschüttelt, getrocknet ($Na_2SO_4$) und eingeengt. Das Rohprodukt wird an Kieselgel mit Dichlormethan/Methanol im Verhältnis 98 : 2 chromatographiert und aus Ethylacetat/Pentan kristallisiert. Man erhält so 155 mg (60 % der Theorie) 1,1-Diethyl-3-(6-methyl-12-vinyl-8α-ergolinyl)-harnstoff.

**Beispiel 2**

50 mg (0,12 mMol) 3-(12-Brom-6-methyl-8α-ergolinyl)-1,1-diethylharnstoff werden in 0,3 ml Dimethylformamid gelöst, mit 2,6 mg (0,004 mMol) Bis-(tri-o-tolylphosphin)-palladium-II-chlorid und 50 mg (0,16 mMol) Tributylvinylzinn versetzt und die Mischung 15 Minuten auf 100 °C erhitzt. Nach Aufarbeitung analog Beispiel 1 werden 39 mg (89,2 % der Theorie) 1,1-Diethyl-3-(6-methyl-12-vinyl-8α-ergolinyl)-harnstoff erhalten.

**Beispiel 3**

Analog Beispiel 1 wird hergestellt:

Aus 3-(12-Brom-6-methyl-8α-ergolinyl)-1,1-diethylharnstoff und Tributylzinnacetylen der 3-(12-Ethinyl-6-methyl-8α-ergolinyl)-1,1-diethylharnstoff in 38 % Ausbeute.

Aus 3-(12-Brom-6-methyl-8α-ergolinyl)-1,1-diethylharnstoff und Allyltributylzinn der 3-(12-Allyl-6-methyl-8α-ergolinyl)-1,1-diethylharnstoff in 46 % Ausbeute.

**Beispiel 4**

Analog Beispiel 2 wird hergestellt:

Aus 3-(12-Brom-2,3β-dihydro-6-methyl-8α-ergolinyl)-1,1-diethylharnstoff und Tributylvinylzinn der 1,1-Diethyl-3-(2,3β-dihydro-6-methyl-12-vinyl-8α-ergolinyl)-harnstoff in 30 % Ausbeute.

**Beispiel 5**

Zu einer Lösung von 419 mg (1 mMol) 3-(12-Brom-6-methyl-8α-ergolinyl)-1,1-diethylharnstoff und 36 mg (0,05 mMol) 1,1′-Bis-diphenylphosphinoferrocenpalladium-II-chlorid in 120 ml Tetrahydrofuran wird eine Lösung von 10 mMol Phenylzinkchlorid gegeben, die aus 10 ml einer 1 molaren Zinkchlorid-Lösung in Tetrahydrofuran und 5 ml einer 2 molaren Phenyllithium-Lösung in Benzol/Diethylether im Verhältnis 75 : 25 bereitet wird. Nach 17 Stunden Rühren bei Raumtemperatur unter Argonatmosphäre wird die Reaktionsmischung unter Eiskühlung mit 2 n Salzsäure angesäuert, mit 25 %iger Ammoniaklösung alkalisch gestellt und mit Dichlormethan ausgeschüttelt. Die organische Phase wird getrocknet ($Na_2SO_4$) eingeengt, das Rohprodukt an Kieselgel mit Dichlormethan/Triethylamin im Verhältnis 99,5 : 0,5 chromatographiert und aus Ethylacetat kristallisiert. Es werden 350 mg (84,1 % der Theorie) 1,1-Diethyl-3-(6-methyl-12-phenyl-8α-ergolinyl)-harnstoff erhalten.

$[\alpha]_D = +31°$ (0,5 % in Chloroform)

**Beispiel 6**

Eine Lösung von 419 mg (1 mMol) 3-(12-Brom-6-methyl-8α-ergolinyl)-1,1-diethylharnstoff in 4 ml Dimethylformamid wird mit 134 mg (1,1 mMol) Benzolboronsäure, 36 mg (0,05 mMol) 1,1′-Bis-diphenylphosphinoferrocenpalladium-II-chlorid und 0,42 ml Triethylamin versetzt. Nach 2 Stunden Rühren bei 100 °C unter Argonatmosphäre wird analog Beispiel 5 aufgearbeitet. Es werden 320 mg (77 % der Theorie) 1,1-Diethyl-3-(6-methyl-12-phenyl-8-α-ergolinyl)-harnstoff erhalten.

$[\alpha]_D = +31°$ (0,5 % in Chloroform)

**Beispiel 7**

Analog Beispiel 5 wird hergestellt:

Aus 3-(2-Brom-6-methyl-8α-ergolinyl)-1,1-diethylharnstoff, Phenylzinkchlorid und 1,3-Bis-diphenylphosphinopropannickel-II-chlorid als Katalysator der 1,1-Diethyl-3-(6-methyl-2-phenyl-8α-ergolinyl)-harnstoff in 50 % Ausbeute.

$[\alpha]_D = +5°$ (0,5 % in Chloroform)

**Beispiel 8**

Analog Beispiel 6 wird hergestellt:
Aus 3-(2-Brom-6-methyl-8α-ergolinyl)-1,1-diethylharnstoff und 4-Methoxy-phenylboronsäure der 1,1-Diethyl-3-[2-(4-methoxyphenyl)-6-methyl-8α-ergolinyl)-harnstoff in 45 % Ausbeute.
$[\alpha]_D = + 10$ ° (0,5 % in Chloroform)

**Beispiel 9**

Zu einer Lösung von 841 mg (10 mMol) Thiophen in 10 ml Tetrahydrofuran werden bei 0 °C 6,25 ml (10 mMol) n-Butyllithium (1,6 molar in Hexan) gegeben. Nach 4 Stunden Rühren bei 0 °C unter Argonatmosphäre werden 11 ml (11 mMol) Zinkchlorid (1 molar in Tetrahydrofuran) hinzugefügt und eine weitere Stunde bei Raumtemperatur gerührt. Die erhaltene Lösung von 2-Thienylzinkchlorid wird zu einer Lösung von 419 mg (1 mMol) 3-(2-Brom-6-methyl-8α-ergolinyl)-1,1-diethylharnstoff und 27 mg (0,05 mMol) 1,3-Bis-diphenylphosphinopropannickel-II-chlorid in 20 ml Tetrahydrofuran gegeben. Nach 48 Stunden Rühren bei Raumtemperatur und Argonatmosphäre wird analog Beispiel 5 aufgearbeitet. Es werden 135 mg (32,2 % der Theorie) 1,1-Diethyl-3-[6-methyl-2-(2-thienyl)-8α-ergolinyl]-harnstoff erhalten.

**Beispiel 10**

Analog Beispiel 9 wird hergestellt:
Aus 3-(12-Brom-6-methyl-8α-ergolinyl)-1,1-diethylharnstoff, 2-Thienylzinkchlorid und 1,1′-Bis-diphenylphosphinoferrocenpalladium-II-chlorid als Katalysator der 1,1-Diethyl-3-[6-methyl-12-(2-thienyl)-8α-ergolinyl]-harnstoff in 55 % Ausbeute.
Aus 3-(2-Brom-6-methyl-8α-ergolinyl)-1,1-diethylharnstoff, 2-Furylzinkchlorid und 1,3-Bis-diphenylphosphinopropannickel-II-chlorid als Katalysator der 1,1-Diethyl-3-[2-(2-furyl)-6-methyl-8α-ergolinyl]-harnstoff in 49 % Ausbeute.

**Beispiel 11**

419 mg (1 mMol) 3-(2-Brom-6-methyl-8α-ergolinyl)-1,1-diethylharnstoff und 27 mg (0,05 mMol) 1,3-Bis-diphenylphosphinopropannickel-II-chlorid werden in 10 ml Diethylether/Tetrahydrofuran im Verhältnis 1 : 1 suspendiert und mit 0,66 ml Methylmagnesiumbromid (3 molar in Diethylether) versetzt. Die Mischung wird 6 Stunden unter Rühren und Argonatmosphäre zum Rückfluß erhitzt und analog Beispiel 5 aufgearbeitet. Das Rohprodukt wird an Kieselgel mit Dichlormethan/Methanol im Verhältnis 98 : 2 chromatographiert und aus Ethylacetat/Pentan kristallisiert. Es werden 184 mg (52 % der Theorie) 1,1-Diethyl-3-(2,6-dimethyl-8α-ergolinyl)-harnstoff erhalten.
$[\alpha]_D = + 13,7$ (0,5 % in Chloroform)

**Beispiel 12**

Eine Lösung von 3,3 g (7,88 m Mol) 3-(2-Brom-6-methyl-8α-ergolinyl)-1,1-diethylharnstoff und 347 mg (0,48 mMol) 1,1′-Bis-diphenylphosphinoferrocenpalladium-II-chlorid in 30 ml Tetrahydrofuran wird mit 15,8 ml 3n-Natronlauge und 17,3 ml Triethylboran (1 molar in Tetrahydrofuran) versetzt. Nach 3 Stunden Rühren unter Rückfluß und Argonatmosphäre wird analog Beispiel 5 aufgearbeitet. Das Rohprodukt wird an Kieselgel mit Dichlormethan/Methanol im Verhältnis 97,5 : 2,5 chromatographiert und aus Ethylacetat kristallisiert. Es werden 888 mg (30,6 % der Theorie) 1,1-Diethyl-3-(2-ethyl-6-methyl-8α-ergolinyl)-harnstoff erhalten.
$[\alpha]_D = + 25$ ° (c = 0,22 in Pyridin)

**Beispiel 13**

Analog Beispiel 12 wird hergestellt:
Aus 1,1-Diethyl-3-(2-jod-6-methyl-8α-ergolinyl)-harnstoff der 1,1-Diethyl-3-(2-ethyl-6-methyl-8α-ergolinyl)-harnstoff in 28 % Ausbeute. $[\alpha]_D = + 25$ ° (c = 0,22 in Pyridin)

**Beispiel 14**

Analog Beispiel 6 wird hergestellt:
Aus 3-(13-Brom-6-methyl-8α-ergolinyl)-1,1-diethylharnstoff der 1,1-Diethyl-3-(6-methyl-13-phenyl-8α-ergolinyl)-harnstoff in 62 % Ausbeute.

**Patentansprüche**

1.) Verfahren zur Herstellung von 2′-, 12′- oder 13′-substituierten 3-(8′α-Ergolinyl)-1,1-diethylharnstoffen der allgemeinen Formel I und deren Säureadditionssalzen

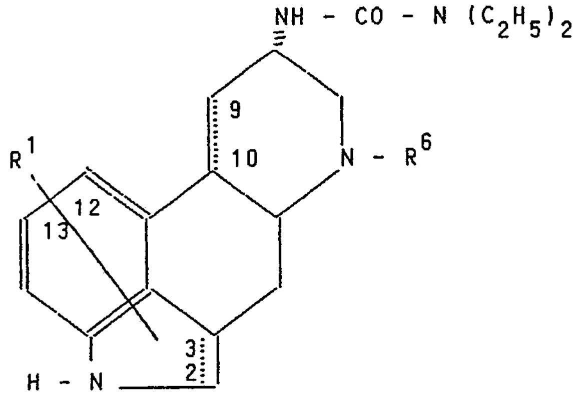

I

worin
$R^1$ $C_{1-10}$-Alkyl, $C_{2-10}$-Alkenyl, $C_{2-10}$-Alkinyl, gegebenenfalls substituiertes Phenyl oder Hetaryl bedeutet,
$R^6$ $C_{1-4}$-Alkyl und
$C_2$- -$C_3$ und $C_9$- -$C_{10}$ eine Einfach oder Doppelbindung bedeuten und $C_2$- -$C_3$ eine Doppelbindung ist, wenn $R^1$ in 2-Stellung steht, dadurch gekennzeichnet, daß man eine Verbindung der allgemeinen Formel II

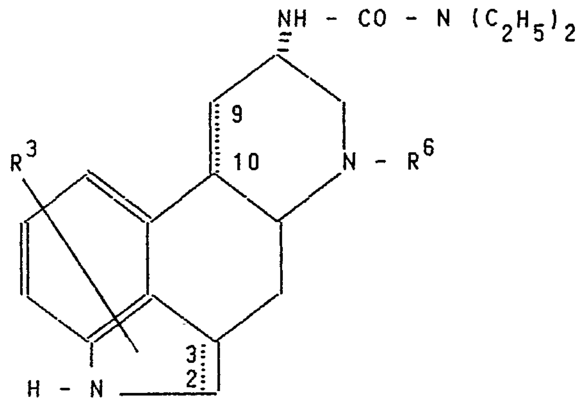

II

worin $C_2$- -$C_3$, $C_9$- -$C_{10}$ und $R^6$ die obige Bedeutung haben und $R^3$ Halogen ist, in Gegenwart eines Nickel- oder Palladiumkatalysators umsetzt mit einer metallorganischen Verbindung der allgemeinen Formel III

$R^1$ - Me - $X_n$ III

worin
$R^1$ die obige Bedeutung hat,
Me ein Metallatom,
X Halogen, Hydroxy oder $C_{1-4}$-Alkyl und
n 1 bis 3 bedeutet und gegebenenfalls anschließend die Säureadditionssalze bildet.

2.) Verbindungen der allgemeinen Formel Ia

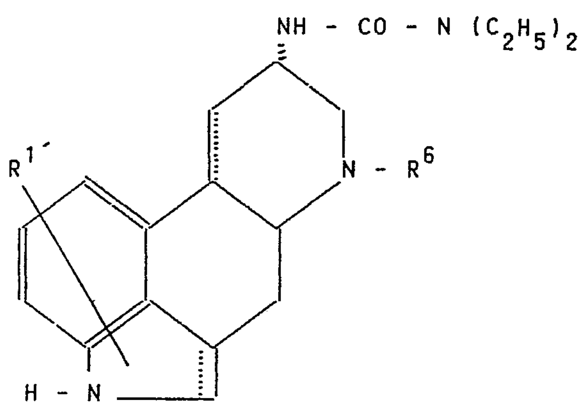

Ia

worin
$C_2$- -$C_3$, $C_9$- -$C_{10}$ und $R^6$ die obige Bedeutung haben und $R^{1'}$ in 12- und 13-Stellung die Bedeutung von $R^1$ hat mit Ausnahme von $C_{1-10}$-Alkyl und $R^{1'}$ in 2-Stellung gegebenenfalls substituiertes Phenyl oder Hetaryl ist, sowie deren Säureadditionssalze.

3.) Arzneimittel auf Basis der Verbindungen gemäß Anspruch 2 und 4.

4.) 1,1-Diethyl-3-(6-methyl-12-vinyl-8α-ergolinyl)-harnstoff

1,1-Diethyl-3-(6-methyl-12-ethinyl-8α-ergolinyl)-harnstoff
1,1-Diethyl-3-(6-methyl-12-phenyl-8α-ergolinyl)-harnstoff
1,1-Diethyl-3-(6-methyl-2-phenyl-8α-ergolinyl)-harnstoff
1,1-Diethyl-3-[6-methyl-2-(2-thienyl)-8α-ergolinyl]-harnstoff